# EUROPEAN PATENT SPECIFICATION

(11) **EP 0 213 933 B1**
(45) Date of publication and mention of the grant of the patent: **03.08.1994**
(21) Application number: 86306648.6
(22) Date of filing: 28.08.1986
(51) Int. Cl.: C07D 207/448

(54) **Maleimide manufacture**
Herstellung von Maleimid
Préparation de maléimide

(30) Priority: 28.08.1985 JP 187437/85
(43) Date of publication of application: 11.03.1987
(73) Proprietor: NIPPON SHOKUBAI CO., LTD., Chuo-ku, Osaka-shi, Osaka-fu 541 (JP)
(72) Inventor: Kishino, Kazuo, Himeji-shi Hyogo-ken (JP); Kita, Yuichi, Akashi-shi Hyogo-ken (JP); Sakamoto, Kentaro, Ibo-gun Hyogo-ken (JP); Baba, Masao, Himeji-shi Hyogo-ken (JP); Nakagawa, Yoichi, Takarazuka-shi Hyogo-ken (JP)
(74) Representative: Sheard, Andrew Gregory

(56) References cited:
- GB-A- 1 041 027
- US-A- 2 444 536
- PATENT ABSTRACTS OF JAPAN, unexamided applications, C section, vol. 2, no. 105, August 30, 1978 THE PATENT OFFICE JAANESE GOVERNMENT page 1940 C 78 * Kokai-no. 53-68 770 (IHARA CHEMICAL KOGYO K.K.) *

## Description

This invention relates to a method for the manufacture of maleimides, by subjecting maleinamic acids in an organic solvent capable of forming an azeotrope with water in the presence of an acid catalyst to ring-closure imidation, which method is characterized by causing a layer containing an acid catalyst which has at least once participated in the reaction to be used again as a catalyst in the reaction thereby enabling maleimides to be produced in an increased yield.

Research on methods for the production of maleimides has long been under way. The most popular method of them all effects the production of maleimides by the dehydration cyclization of maleinamic acids with a dehydrating agent such as acetic anhydride. One version of this method is disclosed in US-A-2,444,536. This method effects the production of maleimides by causing maleic anhydride to react upon amines thereby forming maleinamic acids and dehydration cyclizing and, at the same time, imidating the maleinamic acids in the presence of acetic anhydride and sodium acetate. This method, however, has the disadvantage that the imidation requires expensive acetic anhydride to be used in at least an equivalent relative to the maleinamic acid and the separation and recovery of the maleimide formed from the imidation reaction solution necessitates use of a large volume of water and, as a result, entails disposal of a large amount of an acetic acid-containing effluent at great expense. Thus, this method may well be considered too expensive for commercial production of maleimides.

Further, as disclosed in the specification of Japanese Patent Laid-open SHO 53(1978)-68,770, there is a method which comprises causing maleic anhydride to react with an amine compound in an organic solvent and subjecting the consequently produced maleinamic acid, without being subsequently isolated, to a dehydration ring-closure reaction in the coexistence of an aprotic polar solvent such as dimethyl formamide or dimethyl sulphoxide and an acid catalyst. This method cannot be called satisfactory because it entails various problems such as, for example, the high production cost of a maleimide because of ample use of such an aprotic polar solvent as dimethyl formamide which is expensive and toxic,the inevitable degeneration and heavy loss of the solvent such as dimethyl formamide by the action of an acid catalyst to be used in the reaction, and the difficult removal of the aprotic polar solvent from the produced maleimide because of the high boiling point of the solvent.

In the specification of GB-A-1,041,027 is disclosed a method which comprises effecting thermal dehydration ring-closure using as a diluent such a solvent as toluene, xylene, or chlorobenzene which has a boiling point of not less than 80°C in combination with an acid catalyst such as chlorosulphonic acid, p-toluenesulphonic acid, benzenesulphonic acid, orthophosphoric acid, pyrophosphoric acid, or phosphorous acid and causing the water which is produced during the reaction to be expelled from the system by distillation in the form of an azeotrope with the solvent. This method is excellent in the sense that it obviates the necessity for abundantly consuming such an expensive dehydrating agent as acetic anhydride and further that the produced maleimide is easily separated and recovered.

This method, however, is not economically satisfactory as a commercial means of production because it requires the use of a relatively large amount of such an expensive acid catalyst as chlorosulphonic acid, p-toluenesulphonic acid, benzenesulphonic acid, orthophosphoric acid, pyrophosphoric acid, or phosphorous acid and further because maleimides are produced in a poor yield.

The methods which are now in use for the production of maleimides still have problems and are not satisfactory for the purpose of commercial application.

EP-A-165574 discloses a method for the production of maleimides, which comprises subjecting maleinamic acids to ring-closure imidation in an organic solvent capable of forming an azeorope with water in the presence of an acid catalyst at a temperature in the range of 120° to 250°C while removing the formed water in the form of an azeotrope with said organic solvent and thereafter purifying the produced maleimides.

An object of this invention, therefore, is to provide a method for manufacturing inexpensively maleimides of high purity by a safe and simple procedure.

According to the invention, there is provided a method for the manufacture of maleimides represented by the general formula (I): 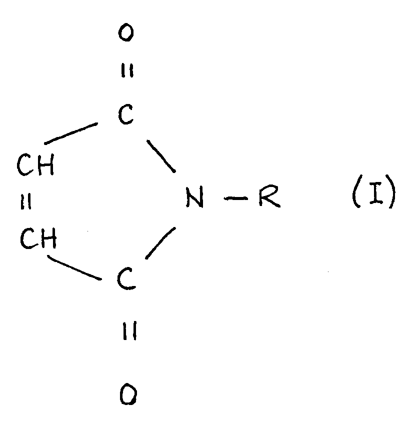
wherein R denotes a member selected from alkyl groups of 1 to 20 carbon atoms, phenyl, benzyl, cyclohexyl, pyridyl, and quinolyl groups, and any of these groups having halogen, methoxyphenyl, ethoxyphenyl tolyl, xylyl, ethylphenyl, carboxyl, or nitro substituents, comprising the steps of:
(i) heating the corresponding maleinamic acid of the formula (II) 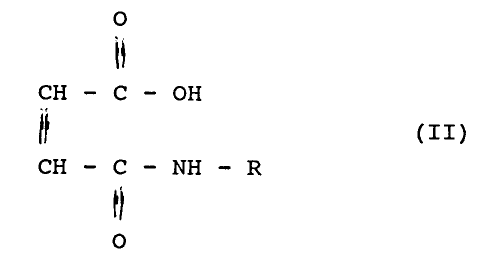 (wherein R is defined as indicated above) in an organic solvent capable of forming an azeotrope with water while
(ii) causing the water formed during the course of the reaction to be removed from the organic solvent, thereby
(iii) effecting ring closure of the maleinamic acid, the acid catalyst comprising at least one acid selected from sulphuric acid, sulphuric anhydride, P-toluenesulphonic acid, orthophosphoric acid, metaphosphoric acid, and pyrophosphoric acid, characterised in that:
   (a) the organic solvent is a solvent which permits the water formed in the dehydration cyclization to be removed by azeotropic distillation and which is insoluble or immiscible in water and refrains from participating in the reaction;
   (b) the reaction is carried out at a temperature in the range of 120° to 250°C;
   (c) the amount of acid catalyst is in the range of 2 to 400 mol% based on said maleinamic acid; and in that
   (d) the catalyst layer is separated and recovered from the organic solvent layer after completion of the ring-closure step and the acid catalyst layer is heated to a temperature in the range of 100° - 200°C and is re-circulated to a subsequent ring-closure step for repeated use.

We have made a diligent study in search of an economic method for the use of an acid catalyst in the reaction and consequently found that the acid catalyst used in the ring-closure imidation and separated as a catalyst layer from the reaction system after completion of the reaction can be used effectively as a catalyst again in the subsequent cycle of the ring-closure imidation. We have made a surprising finding that when the acid catalyst once used in the reaction is used again in a subsequent cycle of the reaction, the yield of the new reaction is not lowered at all, but rather is notably improved.

This is entirely unexpected from the commonsense point of view; normally when an acid catalyst recovered from a reaction is used again in the subsequent cycle of the reaction, the activity of the acid catalyst is lowered and the yield of the reaction is proportionately lowered (as reported in DE-A-1,934,791, for example).

To be specific, this invention concerns a method for the manufacture of a maleimide comprising heating a maleinamic acid in an organic solvent capable of forming an azeotrope with water in the presence of an acid catalyst while causing the water formed during the course of the reaction to be removed from the reaction system in the form of an azeotrope with the organic solvent thereby effecting ring-closure imidation, which method is characterized by separating and recovering an acid catalyst layer from the organic solvent layer at the end of the ring-clossure imidation and using the recovered acid catalyst layer again in the subsequent cycle of the reaction.

The salient feature of the present invention resides in not merely economizing the use of the acid catalyst but also improving the yield of the reaction by repeatedly using the acid catalyst used for the imidation in the subsequent cycles of the reaction. Optionally, the ring-closure imidation may be carried out in the presence of a metal compound and a stabilizer.

### DESCRIPTION OF THE PREFERRED EMBODIMENTS

The maleinamic acids to be used in this invention are easily obtained generally by the reaction of primary amines with maleic anhydride and have the following general formula I. 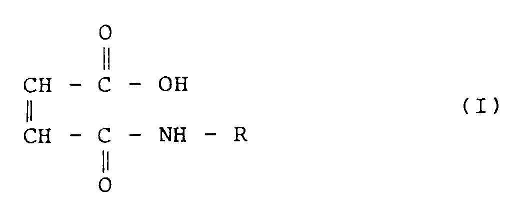
wherein R denotes a member selected from the class consisting of alkyl of 1 to 20 carbon atoms, phenyl, benzyl, cyclohexyl, pyridyl, and quinolyl groups, and any of these groups having halogen, carboxyl, or nitro substituents, and methoxyphenyl, ethoxyphenyl, tolyl, xylol or ethylphenyl. Alkyl groups or phenyl groups are more desirable than the other groups mentioned.

Examples of the primary amine particularly useful as the raw material for the maleinamic acid in this invention include methylamine, ethylamine, n-propylamine, isopropylamine, n-butylamine, sec-butylamine, isobutylamine, tert-butylamine, n-hexylamine, n-dodecylamine, benzylamine, cyclohexylamine, aniline, nitroaniline, aminobenzoic acid, anisidine, ethoxyphenylamine, monochloroaniline, dichloroaniline, toluidines, xylidines, and ethylanilines.

Synthesis of a maleinamic acid proceeds virtually stoichiometrically, so the amine is used in equivalence. For example, however, the maleinamic acid can be synthesized by causing the amine in an amount of 0.8 to 1.5 mols, preferably 0.9 to 1.2 mols, to react upon each mol of maleic anhydride.

The organic solvent capable of forming an azeotrope with water and usable for the purpose of this invention is desirably a solvent which permits the water formed in the dehydration cyclization to be removed by azeotropic distillation and is water insoluble or water immiscible and refrains from participating in the reaction. Typical examples which meet this criteria include benzene, toluene, xylenes, ethylbenzene, isopropylbenzene, cumene, mesitylene, tert-butylbenzene, pseudocumene, trimethylhexane, octane, tetrachloroethane, nonane, chlorobenzene, ethyl cyclohexane, petroleum fractions boiling at 120°to 170°C, m-dichlorobenzene, sec-butylbenzene, p-dichlorobenzene, decane, p-cymene, o-dichlorobenzene, butylbenzene, decahydronaphthalene, tetrahydronaphthalene, dodecane, naphthalene, cyclohexylbenzene, and petroleum fractions boiling at 170°to 250°C. For the purpose of enabling the reaction to proceed smoothly and satisfying the economic conditions of the production, the amount of this solvent to be used in the reaction is desired to fall in the range of 1 to 20 times, preferably 3 to 7 times (by weight) the amount of the maleinamic acids.

The solvent to be used herein is selected on condition that it should possess a boiling point which fits the reaction conditions to be involved, with due consideration paid also to the solubility of the maleimide to be produced, the price of the solvent used, and the ease of handling of the solvent. When the separation of the produced maleimide from the solvent after completion of the reaction is taken into account, it may prove advantageous at times, if not always, to use a solvent having a low boiling point and carry out the reaction under application of pressure.

As the acid catalyst, one member selected from among sulphuric acid, sulphuric anhydride, p-toluenesulphonic acid, orthophosphoric acid, metaphosphoric acid, and pyrophosphoric may be used. The amount of the acid catalyst to be used may be in the range of 2 to 400 mol%, preferably 20 to 200 mol% based on the maleinamic acid to be used.

The reaction may be carried out when necessary in the presence of a metal-containing compound and a stabilizer.

As the acid catalyst added to the reaction system is generally insoluble in the organic solvent to be used in this invention, the reaction system consists substantially assume a state of two layers, i.e., an organic layer and a catalyst layer. This state remains unchanged during and after the reaction. The acid catalyst which has been used in the ring-closure imidation partly has a by-product and a reaction intermediate dissolved therein. It has been confirmed that when this acid catalyst is used in the subsequent cycle of the reaction, therefore, it acts quite effectively in improving the selectivity of the ring-closure imidation.

Since the catalyst layer is a liquid highly viscous at normal room temperature, it should be heated to a temperature in the range of 100° to 200°C, preferably 120° to 180°C, and thereby fluidified enough to ensure ease of handling when it is put to use again in the subsequent cycle of the reaction. Incidentally the catalyst layer, upon being admixed with water, has its viscosity notably lowered. To facilitate the handling of the catalyst layer at normal room temperature, therefore, it is desirable to add 5 to 20% weight of water to the catalyst layer.

When the temperature of the reaction solution is lowered during the separation of the catalyst layer from the organic layer, the impurities dissolved in the organic layer are precipitated possibly to the extent of obscuring the interface between the two layers and rendering the separation of the two layers very difficult. It is, therefore, more advantageous to effect the separation of the two layers at a high temperature. It is desirable that the catalyst layer should be separated from the organic layer immediately after completion of the reaction while the temperature of the solution is still in the range of 120° to 250°C, preferably 130° to 220°C.

The reaction can be carried out when necessary in the presence of a metal-containing compound and a stabilizer. The metal-containing compound so used in the reaction may be selected from oxides, acetates, maleates, succinates, nitrates, phosphates, chlorides and sulphates of at least one metal selected from the group consisting of zinc, chromium, palladium, cobalt, nickel, iron, and aluminium. Among other metal-containing compounds enumerated above, zinc acetate is particularly effective. The amount of the metal-containing compound to be used may be in the range of 0.005 to 0.5mol%, preferably 0.01 to 0.1 mol, as metal, based on the maleinamic acid.

Typical examples of the stabilizer which is effectively used herein include quinones such as methoxybenzoquinone, hydroquinone, and tertbutylhydroquinone; phenols, such as p-methoxyphenol, tert-butyl catechol, alkyl phenols; and alkyl bisphenols; thiodipropionates such as dilauryl thiopropionate; dithiocarbamates such as zinc dimethyl dithiocarbamate and copper dibutyl dithiocarbamate; salicylates; alkylated diphenylamines; phenothiazines such as phenothiazine and methylene blue; and mercaptoimidazoles such as 2-mercaptobenzimidazole, and triphenyl phosphates.

The stabilizer fulfils the part of enabling the maleimide produced by the imidation to retain its quality intact even at the elevated temperature proper to the imidation reaction.

As concerns the amount of the stabilizer to be added, the addition of the stabilizer in a minute amount is not sufficiently effective, whereas the addition thereof in an excess amount is undesirably because it entails the problem of unwanted inclusion of the stabilizer in the final product. The amount of the stabilizer to be added, therefore, is generally in the range of 0.005 to 0.5 mol%, preferably 0.05 to 0.3 mol% per mol of maleinamic acid.

This invention can be worked as follows. First, a solution of maleic anhydride in an organic solvent, and an amine compound added thereto, are left reacting at a temperature not exceeding 150°C, preferably falling in the range of 30° to 120°C, for 15 to 120 minutes to produce maleinamic acid. Then, the resulting reaction solution, with the maleinamic acid left intact therein, and with the acid catalyst or the acid catalyst layer separated from the reaction system added thereto, and further with the metal-containing compound and/or stabilizer added thereto, is heated at a temperature in the range of 120° to 250°C, preferably 130° to 220°C, for 1 to 15 hours and left reacting with the water formed during the reaction expelled out of the reaction system in the form of an azeotrope with the organic solvent. Thus, a maleimide is produced in a high yield.

The present invention described in detail above brings about the following advantages.
(1) The maleimide can be produced in a high yield by causing the acid catalyst once used in the reaction to be used again as a catalyst in a subsequent cycle of the reaction.
(2) The expense of the acid catalyst is substantially negligible because it is used repeatedly in successive cycles of the reaction.
(3) Since the acid catalyst is used repeatedly, the treatment otherwise required for the purpose of rendering the used acid catalyst harmless is virtually unnecessary. The possibility of the used acid catalyst polluting the environment is eliminated.

Owing to advantages (1) to (3) indicated above, this invention permits maleimides to be produced inexpensively, safely and easily.

Now, the present invention will be described more specifically below with reference to working examples.

### Example 1

In a flask provided with a thermometer, a condenser incorporating therein a water separator, a dropping funnel, and a stirrer, 100 g of an oil fraction containing not less than 98% of aromatic hydrocarbons having boiling points in the range of 180° to 220°C and 100 g of maleic anhydride added thereto were heated to a flask interior temperature of 100°C for solution of maleic anhydride.

Then, a solution of 100 g of cyclohexyl amine in 600 g of the solvent was wholly added dropwise under agitation over a period of 1 hour to synthesize a slurry of N-cyclohexyl maleinamic acid in the aforementioned solvent.

Then, the slurry and 60 g of orthophosphoric acid added thereto were heated and stirred at 210°C and, thus, were left reacting for 2 hours, with the water formed during the reaction expelled out of the reaction system by distillation in combination with the solvent. After completion of the reaction, an acid catalyst layer separated to the bottom from the reaction solution at 200°C was separated and removed. Subsequently, the reaction solution was cooled to 60°C and the cooled reaction solution and 100 g of water added thereto were stirred for 30 minutes and washed with water to separate a water layer. This procedure was repeated twice. The organic layer was distilled under a vacuum of 5 mmHg (abs) for expulsion of the solvent.

The contents of the flask, with 0.3 g of copper dibutyldithiocarbamate added anew thereto, were left standing at an inner temperature of 130° to 150°C under a vacuum of 5 mmHg (abs) to effect expulsion of N-cyclohexyl maleimide by distillation over a period, of 30 minutes. As the result, there were obtained 137 g of bright white crystals of N-cyclohexylmaleimide. The maleimide had a purity of 99.8% by weight. The yield of this product was 75.7 mol% based on cyclohexylamine as the raw material.

Then, entirely the same procedure as described above was repeated, except that the acid catalyst layer separated from the reaction system was used in place of orthophosphoric acid. Consequently, there were obtained 153 g of bright white crystals of N-cyclohexylmaleimide. This maleimide had a purity of 99.8% by weight. The yield of this product was 84.7 mol% based on cyclohexylamine as the raw material.

### Example 2

In a flask provided with a thermometer, a condenser incorporating therein a water separator, a dropping funnel, and a stirrer, 100 g of o-xylene and 100 g of maleic anhydride added thereto were heated to a flask interior temperature of 100°C for solution of the maleic anhydride.

Then, a solution of 100 g of cyclohexylamine in 600 g of o-xylene was wholly added dropwise while under agitating over a period of 1 hour to effect synthesis of a slurry of N-cyclohexylmaleinamic acid in o-xylene.

The slurry and 60 g of orthophosphoric acid and 0.1 g of copper dibutyl dithiocarbamate added thereto were heated and stirred at 143°C and left reacting for 7 hours, with the water formed during the reaction expelled out of the reaction system by distillation in combination with the ortho-xylene. After completion of the reaction, an acid catalyst layer separated to the bottom from the reaction solution at 143°C was separated and removed.

Subsequently, the reaction solution was cooled to 60°C and the cooled reaction solution and 100 g of water added thereto were stirred for 30 minutes and washed to separate a water layer. This procedure was repeated twice more. The organic layer was distilled under a vacuum of 10 mmHg (abs) for expulsion of the ortho-xylene.

The contents of the flask, with 0.3 g of copper dibutyl dithiocarbamate added anew thereto, were left standing at an inner temperature of 130° to 150°C under a vacuum of 5 mmHg (abs) to effect expulsion of N-cyclohexyl maleimide by distillation over a period fo 30 minutes. As the result, there were obtained 146 g of bright white crystals of N-cyclohexylmaleimide. This maleimide had a purity of 99.8% by weight. The yield of this product was 80.6 mol% based on cyclohexylamine as the raw material.

Then, entirely the same procedure as described above was repeated, except that the acid catalyst layer separated from the reaction system was used in place of the orthophosporic acid. Consequently, there were obtained 160 g of white crystals of N-cyclohexyl maleimide. This maleimide had a purity of 99.8% by weight. The yield of the product was 88.5 mol% based on cyclohexylamine as the raw material.

### Example 3

In a flask provided with a thermometer, a condenser incorporating therein a water separator, a dropping funnel, and a stirrer, 100 g of o-xylene and 100 g of maleic anhydride added thereto were heated to a flask interior temperature of 100°C for solution of the maleic anhydride.

Then, a solution of 100 g of cyclohexylamine in 600 g of o-xylene was wholly added dropwise while under agitation over a period of 1 hour to synthesize a slurry of N-cyclohexyl maleinamic acid in ortho-xylene.

Then, the slurry and 60 g of orthophosphoric acid and 0.1 g of copper dibutyl dithiocarbamate added thereto were heated and stirred at 143°C and left reacting for 7 hours, with the water formed during the reaction expelled out of the reaction system by distillation in combination with the o-xylene. After completion of the reaction, an acid catalyst layer separated to the bottom from the reaction solution at 143°C was separated and removed.

Entirely the same procedure as described above was repeated, except that the acid catalyst layer separated from the reaction system was used in place of the orthophosphoric acid. The relation between the number of cycles of the reaction and the yield of reaction was studied. The results were as shown in Table 1.

The yield of reaction was determined by analyzing the solution obtained at the end of the reaction by gas chromatography.

**Table 1**

| Number of cycles of reaction | Acid catalyst | Yield (mol% based on amine) |
|---|---|---|
| 1 | Orthophosphoric acid | 84.3 |
| 2 | Acid catalyst layer separated from reaction system | 92.1 |
| 5 | Same as above | 92.8 |
| 10 | Same as above | 93.3 |
| 20 | Same as above | 93.6 |

### Example 4

In a flask provided with a thermometer, a condenser incorporating therein a water separator, a dropping funnel, and a stirrer, a solution of 53 g of maleic anhydride powder in 50 g of xylene was heated to a flask interior temperature of 130°C and a solution of 50 g of aniline in 400 g of xylene was added piecemeal thereto over a period of 30 minutes to synthesize a slurry of N-pheylmaleinamic acid in xylene.

The slurry thus obtained and 10 g of orthophosphoric acid, 0.034 g of zinc acetate, and 0.065 g of p-methoxyphenol added thereto were left reacting at 140°C for 3 hours. After completion of the reaction, the acid catalyst layer was separated from the reaction system. Thereafter, the reaction system was cooled to 30°C, washed with water, and distilled under vacuum to expel the xylene, to afford 85 g of crystals of N-phenylmaleimide. The crystals, on analysis by liquid chromatography, were found to have a purity of 93.1% by weight. The yield of this product was 85.1 mol% based on aniline.

Then, entirely the same procedure as described above was repeated, except that the acid catalyst layer separated from the reaction system was used in place of the orthophosphoric aicd. Consequently, there were obtained 92 g of crystals of N-phenylmaleimide. This maleimide had a purity of 93.6% by weight. The yield of this product was 92.6 mol% based on aniline.

### Example 5

In a flask provided with a thermometer, a condenser incorporating therein a water separator, a dropping funnel, and a stirrer, a solution of 53 g of maleic anhydride in 200 g of chlorobenzene was placed and methylamine gas was blown therein piecemeal at 30°C at a rate of 4.3 x 10⁻³ mol/minute over a period of 2 hours, to afford a white slurry of N-methylmaleinamic acid. Subsequently, this slurry and 22.5 g of orthophosphoric acid, 0.1 g of phenothiazine, and 0.02 g of zinc acetate added thereto were left reacting at 134°C for 2 hours. After completion of the reaction, the acid catalyst layer was separated from the reaction system. The reaction system was cooled to 40°C, filtered, and distilled under a vacuum to expel the chlorobenzene. Consequently, there was obtained 45 g of pale yellow N-methylmaleimide having a purity of 92.1% by weight. The yield of this product was 72.5 mol% based on methylamine as the raw material. Then, entirely the same procedure as described above was repeated except that the acid catalyst layer separated from the reaction system was used in place of the orthophosphoric acid. Consequently, there were obtained 53 g of crystals of N-methylmaleimide. This product had a purity of 93.4% by weight. The yield of this product was 86.4 mol% based on methylamine as the raw material.

### Example 6

The procedure of Example 4 was faithfully repeated, except that the acid catalyst separated from the reaction system was used in the subsequent cycle of the reaction after addition thereto of 10% by weight of water. Consequently, there were obtained 93 g of crystals of N-phenylmaleimide. This maleimide had a purity of 93.4% by weight. The yield of this product was 93.4 mol% based on aniline as the raw material.

## Claims

1. A method for the manufacture of maleimides represented by the general formula (I): 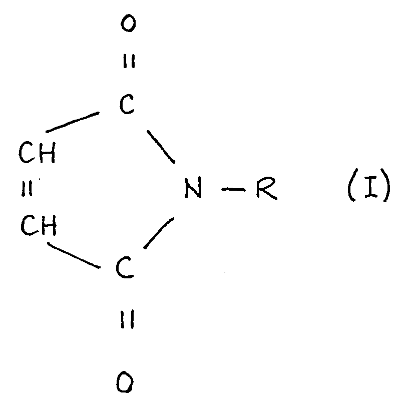 wherein R denotes a member selected from alkyl groups of 1 to 20 carbon atoms, phenyl, benzyl, cyclohexyl, pyridyl, and quinolyl groups, and any of these groups having halogen, carboxyl, or nitro substituents, and methoxyphenyl, ethoxyphenyl, tolyl, xylyl or ethylphenyl comprising the steps of:
(i) heating in the presence of an acid catalyst the corresponding maleinamic acid of the formula (II) 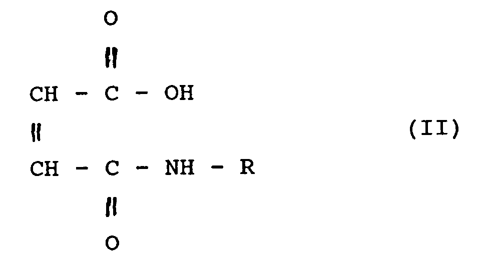 (wherein R is defined as indicated above) in an organic solvent capable of forming an azeotrope with water while
(ii) causing the water formed during the course of the reaction to be removed from the organic solvent, thereby
(iii) effecting ring closure of the maleinamic acid, the acid catalyst comprising at least one acid selected from sulphuric acid, sulphuric anhydride, P-toluenesulphonic acid, orthophosphoric acid, metaphosphoric acid, and pyrophosphoric acid, characterised in that:
(a) the organic solvent is a solvent which permits the water formed in the dehydration cyclization to be removed by azeotropic distillation and which is insoluble or immiscible in water and refrains from participating in the reaction;
(b) the reaction is carried out at a temperature in the range of 120° to 250°C;
(c) the amount of acid catalyst is in the range of 2 to 400 mol% based on said maleinamic acid; and in that
(d) the catalyst layer is separated and recovered from the organic solvent layer after completion of the ring-closure step and the acid catalyst layer is heated to a temperature in the range of 100° - 200°C and is re-circulated to a subsequent ring-closure step for repeated use.

2. A method as claimed in Claim 1 characterised in that the reaction temperature is in the range of 130° to 220°C.

3. A method as claimed in Claim 1 or Claim 2, characterised in that the ring-closure is carried out in the presence of a stabilizer and a compound containing at least one metal which is zinc, chorimium, cobalt, nickel, iron, aluminium or palladium.

4. A method as claimed in Claim 3 characterised in the metal-containing compound is a zinc-containing compound.

5. A method as claimed in Claim 4, characterised in that the metal-containing compound is zinc acetate.

6. A method as claimed in Claim 3, characterised in that the stabilizer is at least one compound selected from quinones, phenols, thio-dipropionic esters, dithiocarbamates, salicylates, alkylated diphenylamines, phenothiazines, mercaptoimidazoles and triphenylphosphates.

7. A method as claimed in any preceding Claim, characterised in that the acid catalyst layer recovered from the organic solvent layer has 5 to 20% by weight of water added thereto before it is re-circulated to the ring-closure step.

## Patentansprüche

1. Verfahren zur Herstellung von Maleinimiden, dargestellt durch die allgemeine Formel (I): 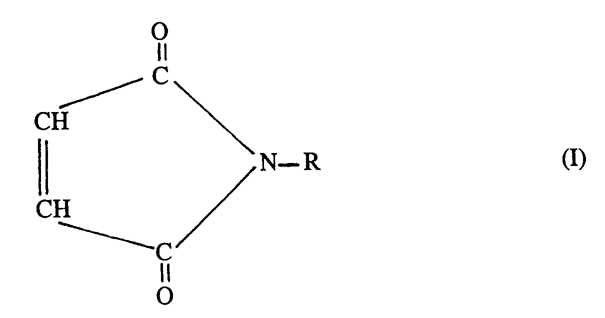 wobei R ein Bestandteil ist, ausgewählt aus Alkylgruppen mit 1 bis 20 Kohlenstoffatomen, Phenyl-, Benzyl-, Cyclohexyl-, Pyridyl- und Chinolylgruppen, und jeder dieser Gruppen, die Halogen-, Carboxyl- oder Nitrosubstituenten aufweisen, und Methoxyphenyl, Ethoxyphenyl, Tolyl, Xylyl oder Ethylphenyl, bei dem man in folgenden Schritten:
(i) das entsprechende Maleinsäuremonoamids der Formel (II) 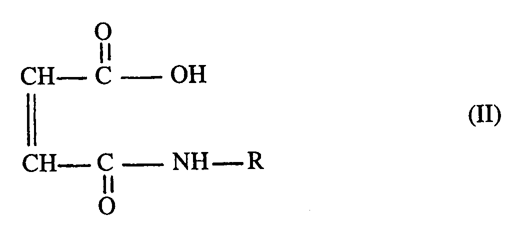 (wobei R definiert ist, wie oben angegeben) in Gegenwart eines Säurekatalysators, der mindestens eine Säure aufweist, die aus Schwefelsäure, Schwefelsäureanhydrid, p-Toluolsulfonsäure, ortho-Phosphorsäure, meta-Phosphorsäure und Pyrophosphorsäure ausgewählt ist, in einem organischen Lösungsmittel, das imstande ist, mit Wasser ein Azeotrop zu bilden, erhitzt,
(ii) das während des Reaktionsverlaufs gebildete Wasser aus dem organischen Lösungsmittel entfernt, und dadurch
(iii) den Ringschluß des Maleinsäuremonoamids vollzieht,
dadurch gekennzeichnet, daß
(a) das organische Lösungsmittel ein Lösungsmittel ist, mit welchem das bei der Dehydratisierungs-Cyclisierung gebildete Wasser durch azeotrope Destillation entfernt werden kann und das in Wasser unlöslich oder unmischbar ist und das nicht an der Reaktion teilnimmt;
(b) man die Reaktion bei einer Temperatur im Bereich von 120° bis 250°C durchführt;
(c) die Menge des Säurekatalysators im Bereich von 2 bis 400 Mol-%, bezogen auf das Maleinsäuremonoamid, liegt; und daß
(d) man die Katalysator-Schicht nach Beendigung des Ringschluß-Schrittes von der organischen Lösungsmittel-Schicht abtrennt und zurückgewinnt und die Säurekatalysator-Schicht auf eine Temperatur im Bereich von 100° bis 200°C erhitzt und zur wiederholten Verwendung für einen nachfolgenden Ringschluß-Schritt rückführt.

2. Verfahren gemäß Anspruch 1, dadurch gekennzeichnet, daß die Reaktionstemperatur im Bereich von 130° bis 220°C liegt.

3. Verfahren gemäß Anspruch 1 oder 2, dadurch gekennzeichnet, daß man den Ringschluß in Gegenwart eines Stabilisators und einer Verbindung, die mindestens ein Metall enthält, das Zink, Chrom, Kobalt, Nickel, Eisen, Aluminium oder Palladium ist, durchführt.

4. Verfahren gemäß Anspruch 3, dadurch gekennzeichnet, daß die metallhaltige Verbindung eine zinkhaltige Verbindung ist.

5. Verfahren gemäß Anspruch 4, dadurch gekennzeichnet, daß die metallhaltige Verbindung Zinkacetat ist.

6. Verfahren gemäß Anspruch 3, dadurch gekennzeichnet, daß der Stabilisator mindestens eine Verbindung ist, die aus Chinonen, Phenolen, Thiodipropionsäureestern, Dithiocarbamaten, Salicylaten, alkylierten Diphenylaminen, Phenothiazinen, Mercaptoimidazolen und Triphenylphosphaten ausgewählt ist.

7. Verfahren gemäß einem der vorhergehenden Ansprüche, dadurch gekennzeichnet, daß man zu der Säurekatalysator-Schicht, die aus der organischen Lösungsmittel-Schicht zurückgewonnen wird, 5 bis 20 Gew.-% Wasser hinzugibt, bevor man sie zum Ringschluß-Schritt rückführt.

## Revendications

1. Procédé de préparation de maléimides représentés par la formule générale (I) : 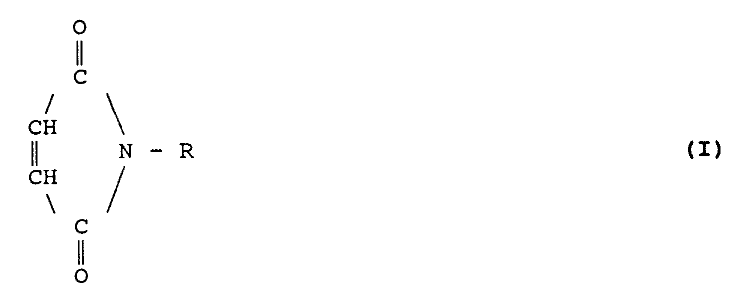 où
R représente un membre choisi parmi les radicaux alcoyle de 1 à 20 atomes de carbone, phényle, benzyle, cyclohexyle, pyridyle et quinoléinyle et l'un quelconque de ces radicaux ayant des substituants halogène, carboxyle ou nitro, et méthoxyphényle, éthoxyphényle, tolyle, xylyle ou éthylphényle, et comprenant les étapes de :
(i) chauffage en présence d'un catalyseur acide, l'acide maléinamique correspondant de formule (II) : 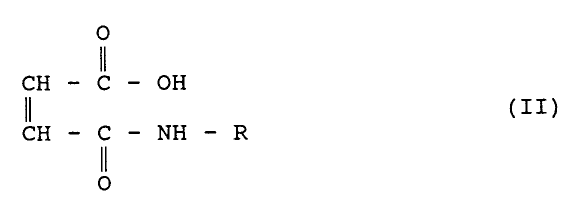 (où R est tel que défini ci-dessus) dans un solvant organique capable de former un azéotrope avec l'eau, tout en
(ii) éliminant du solvant organique l'eau produite au cours de la réaction, afin de
(iii) réaliser la fermeture de cycle de l'acide maléinamique, le catalyseur acide comprenant au moins un acide choisi parmi l'acide sulfurique, l'anhydride sulfurique, l'acide p-toluènesulfonique, l'acide orthophosphorique, l'acide métaphosphorique et l'acide pyrophosphorique, caractérisé en ce que :
(a) le solvant organique est un solvant qui permet à l'eau formée par la cyclisation par déshydratation d'être éliminée par distillation azéotropique et qui est insoluble ou non miscible à l'eau et ne participe pas à la réaction;
(b) la réaction est réalisée à une température dans l'intervalle de 120°C à 250°C;
(c) la quantité du catalyseur acide se situe dans l'intervalle de 2 à 400% en moles sur base de l'acide maléinamique, et
(d) la couche de catalyseur est séparée et récupérée de la couche de solvant organique après achèvement de l'étape de fermeture de cycle et la couche de catalyseur acide est chauffée à une température dans l'intervalle de 100° à 200°C et est recyclée dans une étape de fermeture de cycle suivante, pour un usage répété.

2. Procédé suivant la revendication 1, caractérisé en ce que la température de réaction se situe dans l'intervalle de 130°C à 220°C.

3. Procédé suivant la revendication 1 ou 2, caractérisé en ce que la fermeture de cycle est réalisée en présence d'un agent stabilisant et d'un composé contenant au moins un métal choisi parmi le zinc, le chrome, le cobalt, le nickel, le fer, l'aluminium ou le palladium.

4. Procédé suivant la revendication 3, caractérisé en ce que le composé contenant un métal est un composé contenant du zinc.

5. Procédé suivant la revendication 4, caractérisé en ce que le composé contenant un métal est l'acétate de zinc.

6. Procédé suivant la revendication 3, caractérisé en ce que l'agent stabilisant est au moins un composé choisi parmi les quinones, les phénols, les esters thiodipropioniques, les dithiocarbamates, les salicylates, les diphénylamines alcoylées, les phénothiazines, les mercaptoimidazoles et les triphénylphosphates.

7. Procédé suivant l'une quelconque des revendications précédentes, caractérisé en ce que l'on ajoute à la couche de catalyseur acide récupérée de la couche de solvant organique, de 5 à 20% en poids d'eau, avant qu'elle ne soit recyclée dans l'étape de fermeture de cycle.
